Europäisches Patentamt

European Patent Office

Office européen des brevets

(19)

(11) Publication number: **0 319 168 B1**

(12) **EUROPEAN PATENT SPECIFICATION**

(45) Date of publication of patent specification: **05.08.92**  (51) Int. Cl.5: **A61K 7/32**

(21) Application number: **88310844.1**

(22) Date of filing: **17.11.88**

(54) **Antiperspirant compositions.**

(30) Priority: **20.11.87 US 123086**

(43) Date of publication of application:
**07.06.89 Bulletin 89/23**

(45) Publication of the grant of the patent:
**05.08.92 Bulletin 92/32**

(84) Designated Contracting States:
**AT BE CH DE ES FR GB GR IT LI NL SE**

(56) References cited:
**EP-A- 0 028 853**
**EP-A- 0 203 681**
**EP-A- 0 272 354**
**FR-A- 2 229 753**

(73) Proprietor: **UNILEVER PLC**
**Unilever House Blackfriars P.O. Box 68**
**London EC4P 4BO(GB)**

(84) Designated Contracting States:
**GB**

(73) Proprietor: **UNILEVER N.V.**
**Burgemeester s'Jacobplein 1 P.O. Box 760**
**NL-3000 DK Rotterdam(NL)**

(84) Designated Contracting States:
**BE CH DE ES FR GR IT LI NL SE AT**

(72) Inventor: **Burger, Allan Robert**
**259 Passaic Avenue**
**New Jersey 07417(US)**

(74) Representative: **van Gent, Jan Paulus et al**
**Unilever N.V., Patent Division, P.O. Box 137**
**NL-3130 AC Vlaardingen(NL)**

Rank Xerox (UK) Business Services

**Description**

BACKGROUND AND SUMMARY OF THE INVENTION

The invention relates to antiperspirant compositions.

Numerous compounds have been reported to be active as antiperspirant agents. Of these, basic aluminum halides are perhaps the best known. However, identification of appropriate antiperspirant actives is by no means the final step in devising an effective antiperspirant product. A vehicle must be found which delivers the maximum benefit of the antiperspirant active, which is convenient for everyday use and which is aesthetically pleasing.

Frequently, a vehicle which is otherwise satisfactory is found to deliver an unacceptably low antiperspirant benefit. Moreover, even where a formulation is developed which provides an adequate antiperspirant benefit, it is desirable that the formulation be improved so as to minimise the amount of antiperspirant active which must be used.

Among known antiperspirant formulae is the combination of an antiperspirant active, a solvent comprising volatile silicones and a dispersing agent comprising organomodified clays such as Bentone 38. While this formulation delivers some antiperspirant benefit, it has been desired to develop a product having improved delivery of active so that the benefit is heightened.

Luba, U.S. Patent No. 4,659,571 discloses a compressed powder formulation which consists essentially of an organophilic clay and at least one topical agent which may be an antiperspirant agent such as aluminum zirconium chlorohydrex-gly. The organophilic clay Bentone SD-1 is mentioned in Column 9, and is said in Table 1 to give good results.

European Application EP 272,354 (Unilever NV et al) which relates to this application only in the context of Article 54(3) EPC, describes lotion-type antiperspirant compositions comprising a suspended antiperspirant astringent in an anhydrous liquid medium, the liquid medium comprising a hydrophobically treated day suspending agent, an alcohol which comprises ethanol, isoproponal, or mixtures thereof, and a further liquid less volatile than the alcohol (which can be for example a glycol or a linear volatile silicone), the application also containing a formula for relating the amount of liquid less volatile than the alcohol to the amount of clay in the composition.

European application EP 28,853 (Procter & Gamble) discloses antiperspirant compositions comprising a particulate antiperspirant astringent, a bulking or suspending agent, a volatile silicone, and a non-volatile emollient.

We have now discovered an improved antiperspirant formulation which delivers an improved benefit, as measured in hot room tests. This improved antiperspirant comprises an antiperspirant active compound, a solvent including volatile silicones and a dispersing agent including the organo modified clay, Bentone SD-1. We discovered that when the Bentone SD-1 is used as a dispersing agent in an antiperspirant comprising volatile silicones and active, hot room test results are obtained which are better than for a conventional volatile silicone-based antiperspirant and comparable to the higher efficacy, but cosmetically less desirable, water-based antiperspirants.

The antiperspirant composition of the invention may be used in antiperspirant products taking any of several forms. Specifically, the composition may be used in roll-on, stick, aerosol and pump products. The invention is hereinafter set forth in more detail.

DETAILED DESCRIPTION OF THE INVENTION

The antiperspirant active used in the compositions according to the invention may be any of the known actives. Generally, these comprise the basic aluminum halide compounds and aluminum zirconium chlorhydrates. Typically, the aluminum and zirconium compounds are in the form of astringent salts. The active may optionally be complexed with glycine or other amino acids. Examples of actives for use in the invention include aluminum-zirconium tetrachlorohydrex-glycine, aluminum zirconium penta chlorohydrate, aluminum-zirconium tetrachlorohydrate, aluminum-zirconium trichlorohydrex glycine, aluminum sesquichlorohydrate, aluminum chlorohydrate and aluminum chloride.

In general, the antiperspirant active may be present within the range of from 10 to 25% by weight, preferably 20 to 25%. Preferably the level of active is maximized to yield a high efficacy product. Alternatively, one can take advantage of the improved delivery of the vehicle to minimize the amount of active to be added to the product and yet obtain good antiperspirant protection.

The solvent used in the compositions according to the invention is what is known in the art as "volatile silicones," which are cyclic pentamers and tetramers of dimethyl siloxane (dimethicone).

2

The cyclic pentamers are somewhat less volatile compounds than the cyclic tetramers. Volatile silicones are available, for example, from Dow Chemical as Dow 344 or Dow 345 cyclodimethicone and from Union Carbide Corporation under the designations Volatile Silicone 7207 or 7158. The compositions of the invention generally comprise from 40 to 89%, preferably from 45 to 80%, even more preferably from 50 to 70% by weight of the volatile silicones.

The clay used in the composition according to the invention is Bentone SD-1, a highly dispersible, organomodified clay available from NL Industries, P.O. Box 700, Hightstown, New Jersey 08520. Bentone SD-1 is a clay modified by treatment with a quaternary ammonium compound. Sodium ions in the clay are exchanged for the quaternary ammonium compound. Generally, Bentone SD-1 may be used at from 1 to 10% by weight, preferably from 5 to 10% by weight, and particularly from 8 to 10% by weight.

Although some water may be present in my compositions, preferably the compositions according to the invention are substantially anhydrous, i.e., they contain less than 3% water, and even more preferably the are essentially anhydrous, i.e., contain less than 0.5% water. It is strongly preferred that the compositions of the invention be anhydrous since water tends to dissolve the antiperspirant active. It will be appreciated in this respect that while I have referred to volatile silicone as the "solvent," in fact the active is dispersed throughout the composition, rather than dissolved in it.

Other ingredients common in antiperspirant compositions may be included in the composition according to the invention. For example, particularly for the roll-on formulation, an emollient oil may be present at from 0 to 25% by weight, preferably 1 to 23%, and more preferably 5 to 20%. A suitable emollient oil would be dipropylene glycol methyl ether. Where the composition takes the form of an antiperspirant stick, stearyl alcohol or other waxes may be used within the range of from 10 to 20% by weight. The stick may also include up to 45% by weight talc, preferably from 1 to 40%.

The composition according to the invention may also be in the form of an aerosol concentrate which can be diluted with a propellant such as isobutane or butane. When used in an aerosol, the amount of the components generally are adjusted by dilution with the propellant to fall within the following ranges (percentages are by weight):

antiperspirant active up to 10%, preferably 1 to 10%, more preferably 5 to 10%, and especially 8 to 10;

volatile silicone 0.5 to 25%, preferably 10 to 20%; and

Bentone SD-1 1 to 5%, preferably 1 to 3%.

Where the antiperspirant is in the form of an aerosol, zirconium compounds are not generally appropriate actives.

Regardless of the form of the antiperspirant composition, perfume may be used at a level up to 1%, preferably from 0.05% to 1% by weight.

The following specific example further illustrates the invention.

EXAMPLE I

An antiperspirant roll-on composition in accordance with the present invention was prepared by mixing together and agitating the following components:

|  | % w/w |
|---|---|
| Bentone SD-1 organoclay (NL Industries) | 10.0 |
| Dow 344 cyclodimethicone (Dow) | 52.5 |
| Dowanol DPM dipropylene glycol methyl ether (Dow) | 15.0 |
| Reach AZP-703 micro Zr-Al tetrachlorohydrex (Reheis) | 22.5 |

COMPARATIVE EXPERIMENT I

The roll-on composition of Example I was tested for antiperspirant efficacy in comparison with a "high efficacy" commercial roll-on product, Bristol Meyers' Ultra Ban Extra Strength Roll-on, using 26 females (classified as heavy sweaters) in a standard gravimetric hot room test conducted essentially in accordance with the guidelines published in the Federal Register, October 10, 1978, Part III, Dept. of HEW, Food & Drug Administration, Antiperspirant Dry Products for O-T-C human use, establishment of a monograph, proposed rule.

## RESULTS

**Mean grams of sweat collected**

| | After 3 Applications | After 4 Applications |
|---|---|---|
| Formula of Example I according to the invention | 0.376 | 0.281 |
| Ultra Ban$^R$ | 0.347 | 0.240 |

The mean grams of sweat collected for the composition of Example I and Ultra Ban antiperspirant were not significantly different. The comparatble results obtained with the composition of Example I and the Ultra Ban antiperspirant demonstrate the high efficacy of the present compositions. Furthermore, the product of Example I is more desirably from a cosmetic point of view, since (being silicone based) it lacks the wet/sticky feeling of water based products such as Ultra Ban.

COMPARATIVE EXPERIMENT II

Gravimetric hot room comparison tests of the formula of Example I and a conventional commercially available volatile silicone-based roll-on including Bentone 38 and a comparable level of the active used in Example 1 were conducted essentially in accordance with the published guidelines referred to in EXPERIMENT I. The same procedures were carried out as in EXPERIMENT II, except that there were 29 female participants and the relative humidity in the hot room was 50%. The results were as follows:

**Mean grams of sweat collected**

| | Minutes 41-60 | Minutes 61-80 |
|---|---|---|
| Formula of Example I according to the invention | 0.26 | 0.34 |
| Conventional commercial roll-on | 0.30 | 0.41 |

Based upon the mean weight of sweat collected, the Bentone SD-1-containing roll-on of the invention was found to be significantly more effective than the conventional product.

**Claims**

1. An antiperspirant composition comprising:
   i) from 10 to 25% by weight of an antiperspirant active compound;
   ii) from 40 to 89% by weight of a volatile silicone fluid chosen from cyclic tetromers and pentamers of dimethicone; and
   iii) from 1 to 10% by weight of Bentone SD-1 clay dispersing agent.

2. An antiperspirant composition according to claim 1, in which the antiperspirant active is chosen from aluminium and zirconium compounds in the form of astringent salts.

4

EP 0 319 168 B1

3. An antiperspirant composition according to claim 1 or 2, in which the antiperspirant active is chosen from basic aluminium halide compounds and aluminium zirconium tetrachlorohydrex-glycine.

4. An antiperspirant composition according to claim 1, 2 or 3, in which the antiperspirant active comprises from 20 to 25% by weight of the composition.

5. An antiperspirant composition according to any preceding claim, in which the volatile silicone fluid forms from 45 to 80% by weight of the composition.

6. An antiperspirant composition according to any preceding claim, in which the Bentone SD-1 forms from 5 to 10% by weight of the composition.

7. An antiperspirant composition according to any preceding claim, which further comprises from 0 to 25% by weight of an emollient oil.

8. An antiperspirant composition according to any preceding claim, in the form of a roll-on lotion.

9. An antiperspirant composition according to any of claims 1 to 7, in the form of an antiperspirant stick.

10. An antiperspirant composition according to any of claims 1 to 7, in the form of an aerosol concentrate.

11. An antiperspirant composition according to claim 10, in which the antiperspirant concentrate is further diluted with propellant to provide an aerosol composition in which:
i) the antiperspirant active forms from 1 to 10% by weight of the composition;
ii) the volatile silicone fluid forms from 0.5 to 25% by weight of the composition; and
iii) the Bentone SD-1 forms from 1 to 5% by weight of the composition.

12. An antiperspirant composition according to any preceding claim, which further comprises from 10 to 20% by weight of wax.

13. An antiperspirant composition according to claim 12, wherein the wax is stearyl alcohol.

14. Antiperspirant composition according to any preceding claim, further comprising from 1 to 40% by weight of talc.

**Revendications**

1. Composition antipersirante que comprend:
i) de 10 à 25% en poids d'un composé antiperspirant actif ;
ii) de 40 à 89% en poids d'un fluide siliconique volatil choisi parmi les tetramères et les pentamères cycliques de la diméthicone ; et
iii) de 1 à 10% en poids d'une argile Bentone SD-1 à titre d'agent de dispersion.

2. Composition antiperspirante selon la revendication 1, dans laquelle l'antiperspirant actif est choisi parmi les composés d'aluminium et de zirconium sous forme de sels astringents.

3. Composition antiperspirante selon la revendication 1 ou 2, dans laquelle l'antiperspirant actif est choisi parmi les halogénures basiques d'aluminium et les complexes tetrachlorhydrex d'aluminium-zirconium et de glycine.

4. Composition antiperspirante selon la revendication 1, 2 ou 3, dans laquelle l'antiperspirant actif constitue de 20 à 25% en poids de la composition.

5. Composition antiperspirante selon l'une quelconque des revendications précédentes, dans laquelle le fluide siliconique volatil constitue de 45 à 80% en poids de la composition.

6. Composition antiperspirante selon l'une quelconque des revendications précédentes, dans laquelle la Bentone SD-1 constitue de 5 à 10% en poids de la composition.

5

**7.** Composition antiperspirante selon l'une quelconque des revendications précédentes, qui comprend en outre de 0 à 25% en poids d'une huile émolliente.

**8.** Composition antiperspirante selon l'une quelconque des revendications précédentes, qui est sous forme d'une lotion à distribution par bille.

**9.** Composition antiperspirante, selon l'une quelconque des revendications 1 à 7, qui est sous forme d'un crayon d'antiperspirant.

**10.** Composition antiperspirante selon l'une quelconque des revendications 1 à 7, qui est sous forme d'un concentré pour aérosol.

**11.** Composition antiperspirante selon la revendication 10, dans laquelle le concentré d'antiperspirant est en outre dilué par un propulseur pour former une composition d'aérosol dans laquelle :
i) l'antiperspirant actif constitue de 1 à 10% en poids de la composition ;
ii) le fluide siliconique volatil constitue de 0,5 à 25% en poids de la composition ; et
iii) la Bentone SD-1 constitue de 1 à 5% en poids de la composition.

**12.** Composition antiperspirante selon l'une quelconque des revendications précédentes, qui contient en outre de 10 à 20% en poids d'une cire.

**13.** Composition antiperspirante selon la revendication 12, dans laquelle la cire est l'alcool stéarylique.

**14.** Composition antiperspirante selon l'une quelconque des revendications précédentes, qui contient en outre de 1 à 40% en poids de talc.

**Patentansprüche**

**1.** Antitranspirations-Zusammensetzung, die folgendes umfaßt:
i) 10 bis 25 Gew.-% einer antitranspirationsaktiven Verbindung;
ii) 40 bis 89 Gew.-% einer flüchtigen Siliconflüssigkeit, ausgewählt aus cyclischen Tetrameren und Pentameren von Dimethicon; und
iii) 1 bis 10 Gew.-% Benton SD-1 Tondispersionsmittel.

**2.** Antitranspirations-Zusammensetzung nach Anspruch 1, worin die aktive Antitranspirations-Verbindung ausgewählt ist aus Aluminium- und Zirkoniumverbindungen in der Form von adstringierenden Salzen.

**3.** Antitranspirations-Zusammensetzung nach Anspruch 1 oder 2, worin die aktive Antitranspirations-Verbindung ausgewählt ist aus basischen Aluminium-Halogenidverbindungen und Aluminium-Zirkonium-Tetrachlorohydrex-Glycin.

**4.** Antitranspirations-Zusammensetzung nach Anspruch 1, 2 oder 3, worin die aktive Antitranspirations-Zusammensetzung 20 bis 25 Gew.-% der Zusammensetzung umfaßt.

**5.** Antitranspirations-Zusammensetzung nach einem der vorhergehenden Ansprüche, worin die flüchtige Siliconflüssigkeit 45 bis 80 Gew.-% der Zusammensetzung bildet.

**6.** Antitranspirations-Zusammensetzung nach einem der vorhergehenden Ansprüche, worin das Benton SD-1 5 bis 10 Gew.-% der Zusammensetzung bildet.

**7.** Antitranspirations-Zusammensetzung nach einem der vorhergehenden Ansprüche, welche weiterhin 0 bis 25 Gew.-% eines beruhigenden Öls umfaßt.

**8.** Antitranspirations-Zusammensetzung nach einem der vorhergehenden Ansprüche, in Form einer aufrollbaren Lotion.

**9.** Antitranspirations-Zusammensetzung nach einem der Ansprüche 1 bis 7, in Form eines Antitranspirations-Stifts.

**10.** Antitranspirations-Zusammensetzung nach einem der Ansprüche 1 bis 7, in Form eines Aerosol-Konzentrats.

**11.** Antitranspirations-Zusammensetzung nach Anspruch 10, worin das Aerosol-Konzentrat weiter verdünnt wird mit einem Treibmittel um eine Aerosol-Zusammensetzung herzustellen, worin:
   i) die aktive Antitranspirations-Verbindung 1 bis 10 Gew.-% der Zusammensetzung bildet;
   ii) die flüchtige Siliconflüssigkeit 0,5 bis 25 Gew.-% der Zusammensetzung bildet; und
   iii) das Benton SD-1 1 bis 5 Gew.-% der Zusammensetzung bildet.

**12.** Antitranspirations-Zusammensetzung nach einem der vorhergehenden Ansprüche, welche weiterhin 10 bis 20 Gew.-% Wachs umfaßt.

**13.** Antitranspirations-Zusammensetzung nach Anspruch 12, worin das Wachs Stearylalkohol ist.

**14.** Antitranspirations-Zusammensetzung nach einem der vorhergehenden Ansprüche, welche weiterhin 1 bis 40 Gew.-% Talk umfaßt.